# EUROPEAN PATENT SPECIFICATION

(11) **EP 0 659 091 B1**
(45) Date of publication and mention of the grant of the patent: **13.12.2000**
(21) Application number: 93919750.5
(22) Date of filing: 12.08.1993
(51) Int. Cl.: A61M 1/16, A61L 2/00

(54) **SYSTEM FOR PREPARATION OF A MEDICAL SOLUTION, SUCH AS FOR DIALYSIS AND METHOD FOR CLEANSING OF SUCH A SYSTEM**
VORRICHTUNG ZUR HERSTELLUNG EINER MEDIZINISCHEN LÖSUNG, Z.B. FÜR DIE DIALYSE UND REINIGUNGSVERFAHREN EINER SOLCHEN VORRICHTUNG
DISPOSITIF DE PREPARATION D'UNE SOLUTION MEDICALE DE DIALYSE ET PROCEDE DE NETTOYAGE DU DISPOSITIF

(30) Priority: 01.09.1992 SE 9202506
(43) Date of publication of application: 28.06.1995
(73) Proprietor: Gambro Lundia AB, 220 10 Lund (SE)
(72) Inventor: ARVIDSSON, Lars, S-240 10 Dalby (SE); JÖNSSON, Jörgen, S-275 00 Sjöbo (SE); MAKI, Gerry, Cobe Canada Ltd., Scarborough, Ontario M1B 5R1 (CA)
(74) Representative: Asketorp, Göran
(86) International application number: SE9300668
(87) International publication number: WO9405348

(56) References cited:
- WO-A-80/00413
- WO-A-92/10110
- DE-A- 3 447 989
- US-A- 4 158 034

## Description

### TECHNICAL FIELD

The present invention relates to an apparatus for preparation of a medical solution, such as for dialysis, comprising a preferably conventional control monitor with an input for concentrate and a recirculation circuit connectable to said input for chemical and/or heat sterilisation and/or other cleansing of at least the liquid preparation circuit of the control monitor.

The apparatus is primarily intended for dialysis, though it is also suitable for many other purposes, for example for preparation of replacement liquid for hemodiafiltration and hemofiltration.

The invention also relates to a method for sterilising a concentrate suction pipe forming a part of the above mentioned apparatus, as well as a cleansing chamber belonging to the same apparatus.

### BACKGROUND OF THE INVENTION

American Patents 4 158 034 and 4 293 409 describe a system for chemical sterilisation of a dialysis monitor including a recirculation circuit, such that components upstream of the inlet for the sterilisation fluid can also be sterilized. In the known monitor, sterilisation fluid is drawn from a reservoir with the help of a pipe which is also used for sucking up a concentrate during preparation of dialysis fluid. The same system can also be heat sterilized. Thus, the pipe is instead placed in the heat chamber arranged at the start of the system.

In American Patent 4 728 496 the system for a dialysis monitor arranged for heat sterilisation is shown schematically. This system too includes a recirculation circuit which comprises substantially all the components in the monitor before the dialyser as well as a by-pass conduit for closing the circuit. No concentrate suction pipe is shown. In practice however the system is equipped with such a pipe which also in this case is inserted into the heat chamber at the start of the system for sterilization.

More recently use is being made of a removable concentrate suction pipe which is connected to the monitor via a connector on the monitor when concentrate is to be taken from a substantially rigid container or reservoir. Alternatively the same connector can be connected to a flexible bag containing concentrate. With such a system it has been shown to be difficult to effectively cleanse the concentrate suction pipe. This problem is solved with the aid of the present invention.

### DESCRIPTION OF THE INVENTION

The invention thus relates to an apparatus for preparation of a medical solution, such as for dialysis, comprising a control monitor with an input for concentrate and a recirulation circuit connectable to said input for chemical and/or heat sterilisation and/or other cleansing of a least the liquid preparation circuit of the control monitor.

The invention is characterized by a cleansing chamber as defined in Claim 1. In this way the pipe can be cleansed at the same time as the monitor, primarily since the cleansing liquid can flow through both the pipe and the monitor.

Preferably the apparatus according to the invention is provided with a conduit connected, or connectable, to the concentrate input, the free end of which conduit terminates with a connector which is arranged to be able to be connected to either the pipe or a flexible concentrate bag. In this manner the concentrate can be stored either in a substantially stiff container or reservoir or in a flexible concentrate bag, for example consisting of two plastic sheets welded together.

Furthermore, said connector should be arranged to also be directly connectable to an inlet of the recirculation circuit for closing thereof. If at the same time the cleansing chamber is provided with a second connector which is also connectable to said inlet, these connectors can be substantially identical, which simplifies and reduces the costs of the system.

A simple construction is obtained, if the cleansing chamber can be such that it consists of a tube closed at one end and having substantially the same length as the pipe, which pipe is arranged to sealingly close the other end of the chamber. Should the apparatus be adapted for chemical cleansing, the cleansing chamber can form a container at its closed end for a preferably liquid cleansing agent.

The invention further relates to a method for sterilising, or cleansing in another manner, a concentrate suction pipe forming a part of an apparatus of the above described type. This method is characterized in that the pipe is connected to a recirculation circuit inteded for cleansing fluid for flushing of the pipe.

The pipe is preferably connected to an input for concentrates via a conduit connected to the input, the conduit terminating with a connector which is connectable to the pipe so that the pipe may thereafter be introduced into a cleansing chamber which, in turn, is connected to an inlet of the recirculation circuit.

An effective cleansing is obtained when the cleansing liquid is firstly led through the pipe and thereafter along its exterior, or vice versa, for cleansing of both its interior and exterior.

The invention also relates to a cleansing chamber as defined in Claim 8 intended for an apparatus of the above described type for carrying out the method described thereafter.

If the chamber is to be adapted for chemical cleansing, the said closed end has the shape of a container for a cleansing agent.

### BRIEF DESCRIPTION OF THE DRAWINGS

Fig. 1 shows a portion of the front panel of a dialysis monitor with an input for concentrate and an inlet to a recirculation circuit. A conduit having a connector is connected to said input.
Fig. 2 shows a concentrate suction pipe which is arranged to able to be connected to said connector.
In the same manner Fig. 3 shows a flexible concentrate bag which is also connectable to the connector.
Fig. 4 shows a cleansing chamber belonging to the apparatus according to the invention.
Fig. 5 shows a modification of the same chamber.
Fig. 6 shows the flow path during cleansing of said pipe with the help of the chamber according to Fig. 4, though now on a larger scale.
Finally, Fig. 7 shows in somewhat greater detail the apparatus according to the invention connected for cleansing of the concentrate suction pipe.

### DETAILED DESCRIPTION

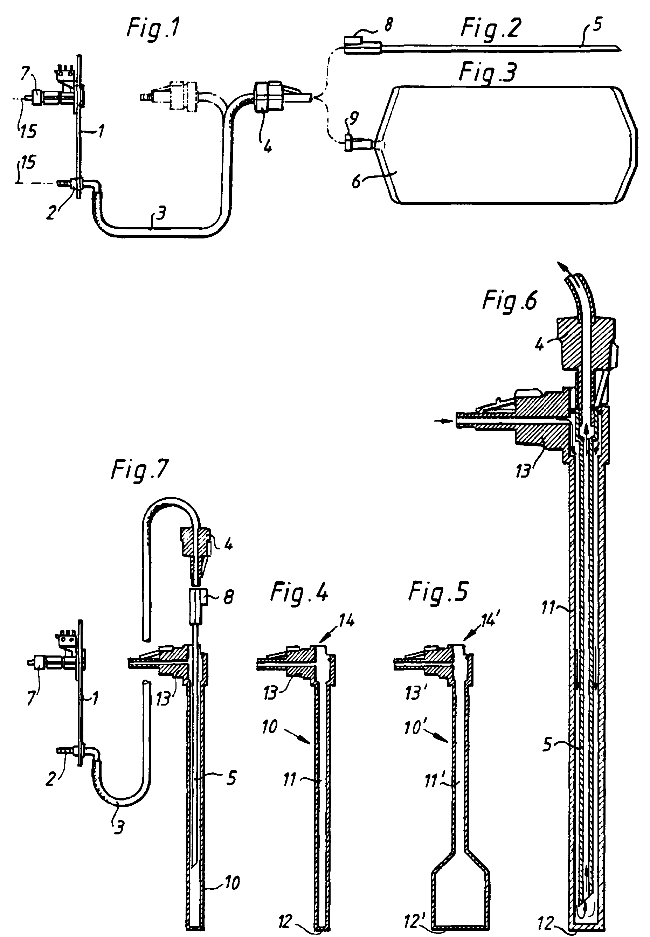

A portion of a monitor panel 1 is shown in Fig. 1, for example the front panel, though any panel could be possible. Reference numeral 2 denotes a concentrate input to which a conduit 3 is connected. This conduit is preferably in the form of a flexible tube. The conduit 3 terminates with a connector 4 which can be connected to three different components, namely the concentrate suction pipe 5 shown in Fig. 2, the flexible concentrate bag 6 shown in Fig. 3 and an inlet 7 to a recirculation circuit 15 within the monitor. The concentrate input 2 is also connected to this circuit 15. The pipe 5 and the bag 6 are provided with connector components 8 and 9 adapted to the connector 4.

Fig. 4 shows a cleansing chamber primarily intended for heat sterilisation and consisting of an elongated tube 11 which is closed at its one end 12 and whose opposite end is provided with a connector 13 which has substantially the same shape as the connector 4 and can therefore be connected to the inlet 7 of the recirculation circuit 15. The cleansing chamber 10 is further provided with an opening 14 through which the pipe 5 can be introduced and which can be sealed with the aid of the connection part 8 of the pipe 5.

Fig. 5 shows a modification of the cleansing chamber according to Fig. 4. It has therefore been denoted by 10'. The difference is that at its closed end it comprises a container 12' for cleansing liquid. The container 12' can either be made in one piece with the tube 11' or be removably attached thereto. The inlet opening for the pipe is denoted by 14' in Fig. 5 and the connector part by 13'.

Fig. 6 hereby shows the cleansing chamber according to Fig. 4 on a somewhat larger scale. As can be seen, in the shown embodiments the cleansing liquid is introduced through the connector part 13 and brought to flow along the outside of the pipe 5 to the lower closed end 12 of the tube 11. The liquid can thereafter flow along the pipe and thereafter through the connector 4 connected to the pipe.

From Fig. 7 it can be seen that the liquid thereafter flows further along the conduit 3 and into the concentrate input 2. The direction of flow can of course also be totally reversed.

The invention is naturally not restricted to solely the above described examples but can be varied within the scope of the appended claims. By the example the shown components can vary in both shape and function.

## Claims

1. Apparatus for preparation of a medical solution, such as for dialysis, comprising a control monitor (1) having a liquid preparation circuit with a concentrate input (2) connected to a preferably dis-connectable concentrate suction pipe (5); and a recirculation circuit (15) connectable to said input (2) for cleansing of at least said liquid preparation circuit of the control monitor by a cleansing agent for chemical and/or heat sterilization, **characterized** by a cleansing chamber (10) included in said recirculation circuit (15) and having a form and size sufficient for receiving said concentrate suction pipe (5), and being arranged for passing said cleansing agent through the interior of the pipe and along its exterior or vice versa for cleansing of both the interior and exterior of said pipe (5).

2. Apparatus according to claim 1, **characterized** by a conduit (3), preferably releasably connected to the concentrate input (2) terminated by a conduit connector (4), adapted to be connected to said pipe (5) or a flexible concentrate bag (6) during normal operation of the monitor and to an inlet (7) of said recirculation circuit (15) for completing the recirculation circuit, during normal cleansing of the monitor.

3. Apparatus according to claim 1 or 2, **characterized** in that the cleansing chamber (10) is provided with a fixed connector (13) adapted for connection to said inlet (7) of the recirculation circuit (15).

4. Apparatus according to any one of the previous claims, **characterized** in that the cleansing chamber (10) consists of a tube (11) having substantially the same length as the pipe (5) so that the pipe is insertable into said tube (11), one end (12) of the tube being closed and the other end being provided with an opening (14) for insertion of the pipe inside the tube.

5. Apparatus according to claim 4, **characterized** in that the cleansing chamber, at its closed end, forms a container (12') or is connected to a container, comprising a preferably liquid cleansing agent.

6. Method for cleansing a concentrate suction pipe (5) forming a part of an apparatus according to any one of the previous claims, said apparatus comprising a control monitor (1) having a liquid preparation circuit with a concentrate input (2) connected to a preferably dis-connectable concentrate suction pipe (5); and a recirculation circuit (15) connectable to said input (2) for cleansing of at least said liquid preparation circuit of the control monitor by a cleansing agent for chemical and/or heat sterilization, **characterized** by placing said pipe (5) into a cleansing chamber included in said recirculation circuit, and passing said cleansing agent firstly through the interior of the pipe (5) and thereafter along its exterior inside said cleansing chamber, or vice versa, for cleansing of both interior and exterior of the pipe.

7. Method according to claim 6, **characterized** in that the pipe (5) is connected to an input (2) for concentrate via a conduit (3) connected to the input, the conduit (3) terminating with a connector (4) which is connectable to the pipe (5), and in that the pipe (5) is introduced into a cleansing chamber (10) which, in turn, is connected to an inlet (7) of the recirculation circuit (15).

8. Cleansing chamber (10) for an apparatus according to anyone of claims 1-5 for carrying out the method according to anyone of claims 6-7, **characterized** in that it comprises an elongated tube (11), one end (12) of the tube being closed and the other end being provided with a first opening (14) and a connector (13) adjacent said other end having a second opening for recirculating a cleansing agent into said chamber.

9. Chamber according to claim 8, **characterized** in that said closed end has the shape of a container (12) for said cleansing agent.

## Patentansprüche

1. Vorrichtung zur Herstellung einer medizinischen Lösung, z.B. für die Dialyse, mit einem Kontrollmonitor (1), der einen Flüssigkeitsherstellungskreislauf mit einem Konzentrateingang (2) hat, welcher mit einem vorzugsweise trennbaren Konzentratsaugrohr (5) verbunden ist; und einem Rückführungskreislauf (15), der mit dem Eingang (2) zum Reinigen mindestens des Flüssigkeitsherstellungskreislaufes des Kontrollmonitors durch ein Reinigungsmittel zur chemischen und/oder Wärmesterilisation verbindbar ist, gekennzeichnet durch eine Reinigungskammer (10), welche in dem Rückführungskreislauf (15) eingeschlossen ist und eine Form und ausreichende Größe zur Aufnahme des Konzentratsaugrohres (5) hat, und die so angeordnet ist, daß das Reinigungsmittel durch das Innere des Rohres und entlang seiner Außenfläche oder umgekehrt zum Reinigen sowohl des Inneren als auch des Äußeren des Rohres (5) hindurchgeht.

2. Vorrichtung nach Anspruch 1, gekennzeichnet durch eine Leitung (3), die vorzugsweise lösbar mit dem Konzentrateingang (2) verbunden ist und in einem Leitungsverbindungsstück (4) endet, wobei die Leitung geeignet ist, mit dem Rohr (5) oder einem flexiblen Konzentratbeutel (6) während des normalen Betriebes des Monitors und mit einem Einlaß (7) des Rückführungskreislaufes (15) zur Vervollständigung des Rückführungskreislaufes während der normalen Reinigung des Monitors verbunden zu werden.

3. Vorrichtung nach Anspruch 1 oder 2, dadurch gekennzeichnet, daß die Reinigungskammer (10) mit einem festen Verbindungsteil (13) versehen ist, das für die Verbindung mit dem Einlaß (7) des Rückführungskreislaufes (15) geeignet ist.

4. Vorrichtung nach einem der vorhergehenden Ansprüche, dadurch gekennzeichnet, daß die Reinigungskammer (10) aus einem Rohr (11), das im wesentlichen dieselbe Länge wie das Rohr (5) hat, besteht, so daß das letztere Rohr in das Rohr (11) einführbar ist, wobei ein Ende (12) des Rohres (11) geschlossen und das andere Ende mit einer Öffnung (14) für die Einführung des Rohres (5) innerhalb des Rohres (11) versehen ist.

5. Vorrichtung nach Anspruch 4, dadurch gekennzeichnet, daß die Reinigungskammer an ihrem geschlossenen Ende einen Behälter (12') bildet oder mit einem Behälter verbunden ist, der ein vorzugsweise flüssiges Reinigungsmittel enthält.

6. Verfahren zum Reinigen eines Konzentratsaugrohres (5), das einen Teil einer Vorrichtung nach einem der vorhergehenden Ansprüche bildet, wobei die Vorrichtung einen Kontrollmonitor (1) aufweist, der einen Flüssigkeitsherstellungskreislauf mit einem Konzentrateingang (2) hat, der mit einem vorzugsweise trennbaren Konzentratsaugrohr (5) verbunden ist; und einen Rückführungskreislauf (15) aufweist, der mit dem Eingang (2) zum Reinigen mindestens des Flüssigkeitsherstellungskreislaufes des Kontrollmonitors durch ein Reinigungsmittel zur chemischen und/oder Wärmesterilisation verbindbar ist, gekennzeichnet durch das Anordnen des Rohres (5) in einer Reinigungskammer, die in dem Rückführungskreislauf enthalten ist, und durch Führen des Reinigungsmittels zuerst durch das Innere des Rohres (5) und danach entlang seinem Äußeren innerhalb der Reinigungskammer, oder umgekehrt, um sowohl das Innere als auch das Äußere des Rohres zu reinigen.

7. Verfahren nach Anspruch 6, dadurch gekennzeichnet, daß das Rohr (5) mit einem Eingang (2) für Konzentrat über eine Leitung (3) verbunden ist, welche mit dem Eingang verbunden ist, wobei die Leitung (3) in einem Verbindungsstück (4) endet, das mit dem Rohr (5) verbindbar ist, und dadurch, daß das Rohr (5) in eine Reinigungskammer (10) eingeführt wird, die ihrerseits mit einem Einlaß (7) des Rückführungskreislaufes (15) verbunden ist.

8. Reinigungskammer (10) für eine Vorrichtung nach einem der Ansprüche 1 bis 5 zur Durchführung des Verfahrens nach einem der Ansprüche 6 bis 7, dadurch gekennzeichnet, daß sie ein verlängertes Rohr (11) aufweist, wobei ein Ende (12) des Rohres geschlossen ist und das andere Ende mit einer ersten Öffnung (14) und einem Verbindungsstück (13) neben dem anderen Ende versehen ist, welches eine zweite Öffnung zur Rückführung eines Reinigungsmittels in die Kammer hat.

9. Kammer nach Anspruch 8, dadurch gekennzeichnet, daß das geschlossene Ende die Form eines Behälters (12) für ein Reinigungsmittel hat.

## Revendications

1. Dispositif de préparation d'une solution médicale, par exemple pour dialyse, comprenant un moniteur de contrôle (1) ayant un circuit de préparation de liquide avec une entrée de concentré (2) connectée à un tube d'aspiration de concentré de préférence déconnectable (5) ; et un circuit de recirculation (15) connectable à ladite entrée (2) pour nettoyage d'au moins ledit circuit de préparation de liquide du moniteur de contrôle au moyen d'un agent de nettoyage pour stérilisation chimique et/ou thermique, caractérisé en ce qu'une chambre de nettoyage (10) est incluse dans ledit circuit de recirculation (15), elle a une forme et une dimension suffisantes pour recevoir ledit tube d'aspiration de concentré (5) et elle est agencée pour faire circuler ledit agent de nettoyage à l'intérieur du tube et le long de sa surface extérieure, ou vice versa, pour nettoyage à la fois de l'intérieur et de l'extérieur dudit tube (5).

2. Dispositif selon la revendication 1, caractérisé en ce qu'il comprend un conduit (3), de préférence raccordé de façon séparable à l'entrée de concentré (2) et terminé par un connecteur de conduit (4) prévu pour être raccordé audit tube (5) ou à une poche souple de concentré (6) pendant le fonctionnement normal du moniteur, et à une entrée (7) dudit circuit de recirculation (15) afin de compléter le circuit de recirculation, pendant le nettoyage normal du moniteur.

3. Dispositif selon la revendication 1 ou 2, caractérisé en ce que la chambre de nettoyage (10) est pourvue d'un connecteur fixe (13) agencé pour connexion à ladite entrée (7) du circuit de recirculation (15).

4. Dispositif selon une quelconque des revendications précédentes, caractérisé en ce que la chambre de nettoyage (10) consiste en un corps tubulaire (11) ayant sensiblement la même longueur que le tube (5) de sorte que le tube est insérable dans ledit corps tubulaire (11), une extrémité (12) du corps tubulaire étant fermée et l'autre extrémité comportant un orifice (14) pour l'insertion du tube à l'intérieur du corps tubulaire.

5. Dispositif selon la revendication 4, caractérisé en ce que la chambre de nettoyage, à son extrémité fermée, définit un récipient (12') ou est connectée à un récipient, contenant un agent de nettoyage de préférence liquide.

6. Procédé de nettoyage d'un tube d'aspiration de concentré (5) faisant partie d'un dispositif selon une quelconque des revendications précédentes, ledit dispositif comprenant un moniteur de contrôle (1) ayant un circuit de préparation de liquide avec une entrée de concentré (2) raccordée à un tube d'aspiration de concentré de préférence déconnectable (5) ; et un circuit de recirculation (15) connectable à ladite entrée (2) pour le nettoyage d'au moins ledit circuit de préparation de liquide du moniteur de contrôle par un agent de nettoyage pour stérilisation chimique et/ou thermique, caractérisé en ce qu'on place ledit tube (5) dans une chambre de nettoyage incluse dans ledit circuit de recirculation et on fait passer ledit agent de nettoyage d'abord dans l'intérieur du tube (5) et ensuite le long de sa surface extérieure, à l'intérieur de ladite chambre de nettoyage, ou vice versa, afin de nettoyer à la fois l'intérieur et l'extérieur du tube.

7. Procédé selon la revendication 6, caractérisé en ce que le tube (5) est raccordé à une entrée (2) de concentré via un conduit (3) raccordé à l'entrée, le conduit (3) se terminant par un connecteur (4) qui est connectable au tube (5), et en ce que le tube (5) est introduit dans une chambre de nettoyage (10) qui est ellemême raccordée à une entrée (7) du circuit de recirculation (15).

8. Chambre de nettoyage (10) pour un dispositif selon une quelconque des revendications 1 à 5 pour la mise en oeuvre du procédé selon une quelconque des revendications 6 et 7, caractérisée en ce qu'elle comprend un corps tubulaire allongé (11), dont une extrémité (12) est fermée et dont l'autre extrémité comporte un premier orifice (14), et un connecteur (13) adjacent à ladite autre extrémité et comportant un deuxième orifice pour la recirculation d'un agent de nettoyage à l'intérieur de ladite chambre.

9. Chambre selon la revendication 8, caractérisée en ce que ladite extrémité fermée a la forme d'un récipient (12) pour ledit agent de nettoyage.
